# EUROPEAN PATENT SPECIFICATION

(11) **EP 1 710 578 B1**
(45) Date of publication and mention of the grant of the patent: **16.09.2009**
(21) Application number: 06007390.5
(22) Date of filing: 07.04.2006
(51) Int. Cl.: G01N 33/487

(54) **Method and apparatus for forming a lipid bilayer membrane**
Verfahren und Vorrichtung zur Herstellung einer Lipid-Doppelschichtmembran
Procédé et appareil de fabrication d'une membrane lipidique bicouche

(30) Priority: 08.04.2005 US 101550
(43) Date of publication of application: 11.10.2006
(73) Proprietor: Charité - Universitätsmedizin Berlin, 10117 Berlin (DE)
(72) Inventor: Pohl, Elena Dr., 16567 Mühlenbeck (DE); Beck, Valeri, 13583 Berlin (DE)
(74) Representative: Bittner, Thomas L.

(56) References cited:
- WO-A-20/05029056
- DE-A1- 10 047 390
- US-A- 5 378 342
- CORONADO R ET AL: "PHOSPHOLIPID BILAYERS MADE FROM MONOLAYERS ON PATCH-CLAMP PIPETTES" BIOPHYSICAL JOURNAL, NEW YORK, US, US, vol. 43, no. 2, August 1983 (1983-08), pages 231-236, XP008066362 ISSN: 0006-3495
- EHRLICH B E: "PLANAR LIPID BILAYERS ON PATCH PIPETTES: BILAYER FORMATION AND ION CHANNEL INCORPORATION" METHODS IN ENZYMOLOGY, ACADEMIC PRESS INC, SAN DIEGO, CA, US, vol. 207, 1992, pages 463-470, XP008066349 ISSN: 0076-6879

## Description

The invention relates to a method for forming a lipid bilayer membrane and an apparatus.

### Background of the invention

The number of membrane proteins identified from genome sequencing and cDNA library searches now greatly exceeds the number of electrophysiologically characterized transport and channel proteins, and identification of their functions based on their transport properties remains a challenge for the future.

Whereas the investigation of small water-soluble proteins exhibits no essential problems, handling of highly hydrophobic membrane proteins still requires advances in methodological approach. A technique for evaluating electrophysiological properties of protein channels, a patch clamp recording of the cell membrane (see Sakmann, B. et al.: Annu. Rev. Physiol 46, 455-472 (1984)), provides good signal resolution, however, it is sometimes difficult to assign the measured channels to specific proteins. Moreover, it is inapplicable for studies of ion channels located on intercellular membranes, for example, in endoplasmic or sarcoplasmic reticulum.

Protein reconstitution into artificial lipid bilayers represents an alternative method allowing the biochemical analysis of defined protein preparations in a precisely known lipid and buffer environment. Since the first description of artificial planar lipid membrane formation (see Mueller, P. et al.: J. Phys. Chem. 67, 534-535 (1963)), two main approaches allowing successful reconstitution of isolated and purified protein have been developed: (i) a "monolayer elevation" method described (see Schindler, H. et al.: Methods Enzymol. 171, 225-253 (1989)) and (ii) membrane formation on glass patch pipettes (see Hanke, W. et al.: Biochim. Biophys. Acta 727, 108-114 (1983)). The first technique uses the finding that monolayers are formed spontaneously at the air-water interface of a proteoliposome suspension. Two monolayers can be combined into a protein-containing bilayer by raising the solution level on both sides of a septum that divides the chamber into two parts (see for example Schindler, H.: FEBS Lett. 122, 77-79 (1980)). At present, this method seems to be the most frequently used approach for studies of purified proteins in artificial lipid bilayers (Saparov, S. M. et al.: J. Biol. Chem. 276, 31515-31520 (2001)).

Disadvantages of this technique include cleaning and preparation of the chamber, as well as hole punching.

The second method implies the formation of planar lipid bilayer membranes at the end of glass patch clamp pipettes. Better current resolution and simple handling have been claimed as advantages of this method. However, the use of this interesting approach appears to have declined in recent years. The reasons include a high membrane fragility and difficulties in control for the membrane quality because membrane capacity and, thus, membrane diameter cannot be measured accurately due to membrane movements inside the patch pipette. Both methods are rather time consuming and require a well-trained researcher.

WO 2005/029056 discloses a method and an apparatus for forming an artificial lipid bilayer membrane. The apparatus comprises a lower solution chamber and an upper solution chamber comprising a small hole in the bottom of the upper solution chamber.

"Phospholipid Bilayers made from Monolayers on Patch-Clamp Pipettes", R. Coronado and R. Latorre, Biophysical Journal, NY, US, vol. 43, no. 2, p. 231-236, discloses a method for producing phospholipid bilayers on the tip of a patch-clamp pipette.

### Summary of the invention

It is therefore an object of the invention, to provide an improved method for protein reconstitution into a lipid bilayer membrane and an apparatus which can be used for an automated lipid bilayer membrane formation, especially for protein reconstitution into an lipid bilayer membrane.

According to an aspect of the disclosed teachings a method for forming a lipid bilayer membrane is provided, the method comprising: a) forming a layer of lipid molecules on a liquid surface at an air-liquid interface between a liquid volume and an air volume; b) moving an aperture from the liquid volume to the air volume through the layer of lipid molecules to form a lipid monolayer membrane at the aperture, the aperture being at an end of a bilayer former; and c) moving the aperture from the air volume to the liquid volume through the layer of lipid molecules to form the lipid bilayer membrane; where an angle of >90° is maintained between the liquid surface and a surface of the aperture during the formation of the lipid monolayer membrane and during the formation of the lipid bilayer membrane.

Another aspect of the disclosed teachings is an apparatus for forming a lipid bilayer membrane, the apparatus comprising: a liquid reservoir operable to receive a liquid volume having an air-liquid interface between the liquid volume and an air volume, a liquid surface of the liquid volume having a layer of lipid molecules on the liquid surface; a bilayer former with an aperture at one end; a mover operable to move the bilayer former; and a controller operable to control the moving of the bilayer former; where the bilayer former and the mover are operable to move said aperture from said liquid volume into said air volume through said layer of lipid molecules on said liquid surface, thereby forming a lipid monolayer membrane in said aperture and are further operable to move the aperture comprising said lipid monolayer membrane from said air volume into said liquid volume through said layer of lipid molecules on said liquid surface, thereby forming a lipid bilayer membrane in said aperture, wherein said bilayer former is operable to maintain an angle >90° between the liquid surface of said aperture during said formation of said lipid monolayer membrane and during said formation of said lipid bilayer membrane.

Further enhancements are provided as listed in the dependent claims.

With the disclosed teachings, an automatic approach is provided which reduces substantially the efforts and allow the usage of one-way material for forming a lipid bilayer membrane, especially for the reconstitution of membrane proteins in artificial membranes.

For example, conventional pipettes with a diameter of about 150 µm to about 300 µm can preferably used as bilayer former. Because such membranes are much larger than membranes formed on the tip of a glass patch pipette (about 0.5 µm to 5 µm), much more protein can be reconstituted. As a result the signal to noise ratio is improved. Albeit the membrane surface is bigger, the amount of proteoliposomes is reduced because less buffer solution is required (about 50 to 100 µl). In contrast to the known glass pipette method, the specific membrane capacitance, C_{spec}, can be measured exactly, because the diameter of the membrane provided can be precisely measured by microscope. Thus, an important criterion for the verification of bilayer formation is fulfilled. An important advantage of the proposed apparatus is worth mentioning. Conventional plastic pipettes and container may be used which are cheap one-way objects and can be easily replaced. No additional expensive devices are necessary for pipette pulling.

### Description of preferred embodiments

The above objectives and advantages of the disclosed teachings will become more apparent by describing in detail preferred embodiments thereof with reference to the attached drawings which show as follows:
- Fig. 1: a schematic view of an apparatus for automated protein reconstitution into lipid bilayer membranes;
- Fig. 2A-2F: a schematic showing the formation of solvent-free bilayers from a preformed lipid monolayer at an aperture of a pipette tip made of a plastic material;
- Fig. 3: current-voltage relationships of UCP2-containing membranes in the absence (circles) or presence (triangles) of ω-6-eicosatrienoic acid (EA), and of membranes containing EA and 0.9 mM ATP (diamonds);
- Fig. 4: gramicidin pore fluctuation in bilayers made from E. coli lipid in the aperture of the pipette tip, where a buffer solution contained 0.5 M KCl at pH 6.8; and
- Fig. 5: a schematic view of an apparatus for automated protein reconstitution into lipid bilayer membranes in a multi-channel set-up.

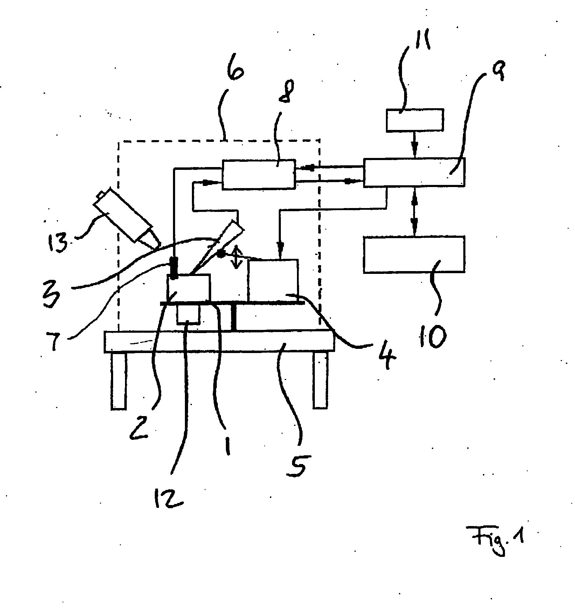

Referring to Fig. 1, an apparatus for forming a lipid bilayer membrane is depicted. A thermostatic unit 1 is provided with a container 2 for receiving a liquid volume. A pipette tip 3 is held by a pipette elevator 4 for moving the pipette tip 3 through a liquid surface of the liquid volume in the container 2. The pipette tip 3 is made of a plastic material. The container 2, and the pipette elevator 4 are located on a vibration isolation table 5. A faraday cage 6 is provided.

A reference electrode 7 is connected to a preamplifier 8. The preamplifier 8 is connected to an amplifier 9 with built-in AD converter. For controlling measures, a Personal Computer 10 is connected to the amplifier 9. In addition, for noise reduction, a power line conditioner 11 connected to the amplifier 9 is provided. The liquid in the container 2 can be stirred by means of a stirrer 12. A light microscope 13 is provided to observe the membrane area formed in the process.

Following, the use of the apparatus depicted in Fig. 1 for lipid membrane formation is described.

### 1. Formation of bilayer membranes from UCP2-containing monolayers

For UCP2 expression, extraction, purification and reconstitution into liposomes human UCP2-containing plasmids were transformed into the bacterial strain BL21 (Novagen) as known from prior art (see Zackova, M. et al.: Biosci. Rep. 22, 33-46 (2002); Jaburek, M. et al.: J. Biol. Chem. 278, 25825-25831 (2003); Jaburek, M. et al.: J. Biol. Chem. 274, 26003-26007 (1999); Jaburek, M. et al.: J. Biol. Chem. 276, 31897-31905 (2001)). Extraction from inclusion bodies and purification of UCP2 followed previously described protocol. A minor modification included the type and the amount of phospholipids used, so that 2.5 mg of the total phospholipids extract from *E.coli* (Sigma) was used *per* mg of inclusion bodies.

The obtained sample of recombinant UCP2 was reconstituted into liposomes using previously described procedures, employing incubation of the protein/lipid mixture in 30 mM K-TES, 80 mM K₂SO₄, 2 mM EDTA, pH 7.2, with Bio-Beads SM-2 (Bio-Rad). The obtained proteoliposomes were stored at -80 °C and thawed the day of experiment. To introduce fatty acids, proteoliposomes were mixed with FA-containing liposomes in required proportions and unilamellar vesicles, containing FA and UCP2 were than extruded through the filter of 100 nm pore diameter 21 times using a small-volume extrusion apparatus (Avestin Inc., Ottawa, Canada).

Bilayer membranes containing UCP2 were formed on the pipette tip 3 (see Fig. 1) having a diameter of about 150 µm to about 300 µm.

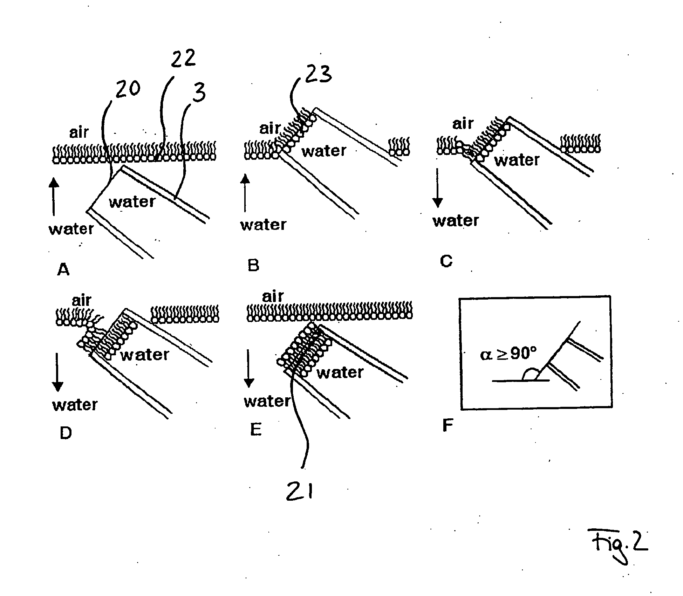

Referring to Fig. 2A-2F, at the beginning of the experiment, the pipette tip 3 was filled with the buffer solution and attached to the pipette elevator 4 manually. An angle >90° is maintained between the water surface and a pipette aperture surface 20 (see Fig. 2F). The pipette tip 3 was therefore flexed. A small plastic one-way container 2 with a volume of 0.75 ml and surface diameter of 12 mm was filled with the proteoliposome suspension, permanently stirred and maintained at 37°C. A bilayer 21 is formed by the transfer of the first one (see Fig. 2A, 2B) and then the second phospholipid monolayer (see Fig. 2C-2E) to the pipette tip 3. The pipette tip 3 and phospholipids are not drawn to scale.

The pipette tip 3 was placed several millimeters under a air-solution surface 22 (see Fig. 2A) and pipette elevator 4 (see Fig. 1) was started. The raising of the pipette tip 3 from the bath led usually to the formation of a first monolayer 23. After achieving a fix point over the air-solution surface 22, the pipette tip 3 was lowered slowly into the solution again. As the pipette tip 3 passes through the water-lipid interface, it becomes coated with a second monolayer of lipid molecules, thereby forming the lipid bilayer 21. Pipette tip 3 elevating and lowering was carried out automatically. Typically the device operated at a frequency of about 0.5 Hz to 1 Hz. It was controlled by an electronic controller, which stopped the up and down movement after membrane formation. Therefore a feedback loop was installed to the analog output of the current amplifier.

The reconstitution of purified recombinant uncoupling protein 2 (UCP2) was described above. The latter lacks the disadvantages of the prior art methods and allows the automatic formation of large membranes at the tip of a pipette. Urgent needs to study UCP2 was inspired by discoveries of a range of mitochondrial inner membrane proteins belonging to the uncoupling protein subfamily (UCP2, UCP3, UCP4, UCP5) by aimed searching of cDNA library. The novel proteins have a high homology to UCP1, which is expressed exclusively in brown adipose tissue. The distribution pattern of UCP2-UCP5 is surprising, reaching from UCP2 transcript, ubiquitously present of in all mammalian tissues, over the preferential distribution of UCP3 in muscles to the mainly brain expressed UCP4 and UCP5. Their molecular transport mechanisms and functions are either unknown or in dispute, despite numerous experiments using reconstituted recombinant proteins, cell cultures and knock out mice.

### 2. Monitoring the capacitance of the membrane, made of UCP2 containing liposomes

To verify the formation of the bilayer 21 (see Fig. 2E), the capacity of the membrane was permanently monitored. For this goal, a triangle input wave with a peak to peak amplitude of 100 mV and a frequency of 10 Hz was applied to the input reference electrode after starting the pipette elevator 4 (see Fig. 1). An electronic scheme, connected to the amplifier 9, was analyzing the output signal from the reference electrode 7. A feedback signal was transmitted to the pipette elevator 4 allowing a continuous cycle of pipette elevation followed by slow pipette lowering if bilayer formation was not indicated by capacitance measurements. In the case of successful membrane formation, the feedback signal stopped the elevation process. Specific capacity, C_{spec}, was calculated with respect to membrane surface area as determined using the light microscope 13. It was equal to 0.95 ± 0.01 µF/cm² for bilayers both with and without protein. These values are similar to the values reported for membranes formed from proteoliposomes. The measured capacity remained constant during the experiment.

### 3. Measurements of the membrane conductivity in presence of ω-6-eicosatrinoic acid

Current-voltage (I-V) characteristics were measured by a patch-clamp amplifier (EPC 10, Heka Elektronik Dr. Schulze GmbH, Germany). For conductance measurements, a ramp voltage signal operating at frequencies of 0.016 Hz was used. Membrane conductance G was determined at zero voltage from a linear fit of voltages in the interval between -50 and 50 mV. For noise reduction the power-line conditioner 11 (see Fig. 1) was used. The apparatus was fixed onto the vibration-free table 5. Data acquisition and processing were performed by Software Pulse (v. 8.65, HEKA Elektronik Dr. Schulze GmbH, Germany).

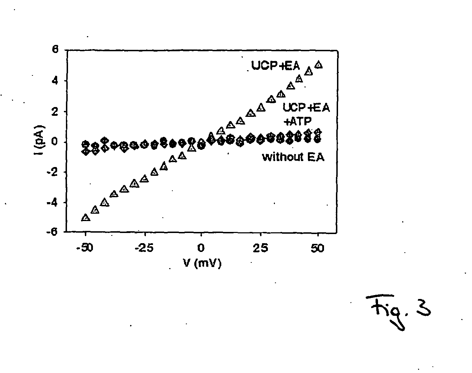

Fig. 3 shows current-voltage relationships of UCP2-containing membranes in the absence (circles) or presence (triangles) of ω-6-eicosatrienoic acid (EA), and of membranes containing EA and 0.9 mM ATP (diamonds). Lipid content was 1,5 mg/ml. The buffer solution contained 50 mM K₂SO₄, 25 mM TES, 0.6 mM EGTA, pH 7.35, and the temperature was 37°C.

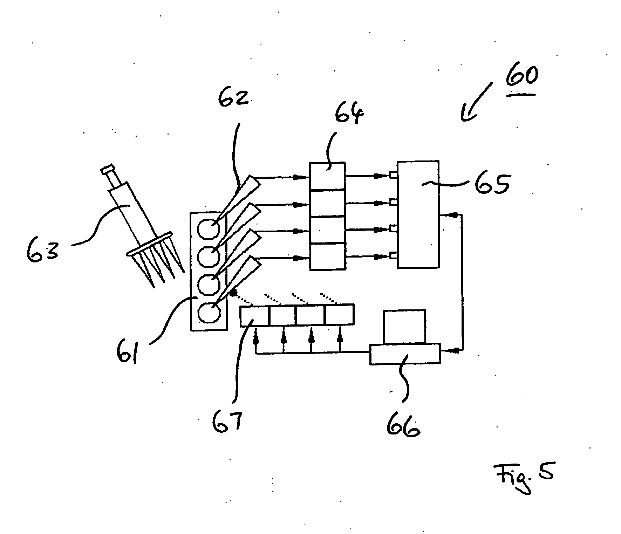

Analogously to previous results with UCP1, the increased UCP2-mediated conductivity in presence of FA was nearly completely inhibited by ATP, if added on both sides of the membrane. Fig. 5 shows results for the inhibition of UCP2-mediated FA-dependent proton transport by ATP. The concentration of UCP2 and ATP were 15 µg/ml and 0.1 mM, respectively.

### 4. Reconstitution of the model peptide gramicidin

In the apparatus (see Fig. 1), the peptide gramicidin (Fluka Chemie GmbH, Buchs, Switzerland) was added from an ethanolic stock solution to the buffer solution. G gramicidin is known to form characteristic voltage-dependent multi-level pore fluctuations in bilayer membranes (see Hladky, S. B. et al.: Nature 5231, 451-453 (1970); Hladky, S. B. et al.: Biochim. Biophys. Acta 274, 294-312 (1972); Bamberg, E. et al.: J. Membr. Biol. 11, 177-194 (1973)). Because gramicidin exhibits these characteristic channel states only in true bilayer membranes, their visulisation provides additional evidence that the pipette is sealed by a single lipid bilayer.

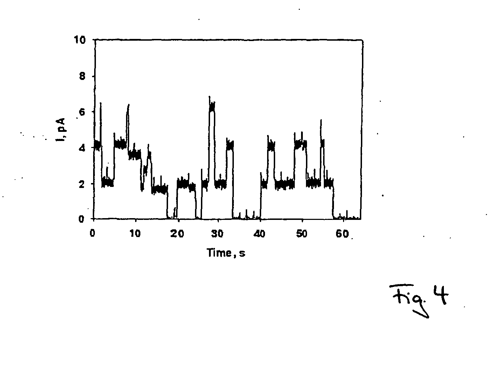

Fig. 4 illustrates the typical gramicidin channel activity in the apparatus described above. A solvent-free membrane monolayer was made by forming a monolayer from the total lipid E. coli extract (Sigma Chemical Co., St. Louis, MO) on top of the buffer solution. Buffer solution contained 0.5 M KCl at pH 6.8. Gramicidin was added at a concentration of 5*10⁻⁷ mg/ml. The holding potential was 100 mV, the temperature was 37°C. Current flow through the bilayer was monitored using the same amplifier (EPC 10, HEKA). The chamber was clamped at virtual ground while the pipette was clamped to the desired holding potential of 100 mV.

With the disclosed teachings an automatic approach is provided which reduces substantially the efforts and allow the usage of one-way material for forming a lipid bilayer membrane, especially for the reconstitution of membrane proteins in artificial membranes. In comparison to known reconstitution methods at least three important changes were made:
(i) Conventional pipettes with a diameter of about 150 µm to about 300 µm were used. Because such membranes are much larger than membranes formed on the tip of a glass patch pipette (about 0.5 µm to 5 µm), much more protein can be reconstituted. As a result the signal to noise ratio is improved. Albeit the membrane surface is bigger, the amount of proteoliposomes is reduced because less buffer solution is required (about 50 to 100 µl). In contrast to the glass pipette method, the specific membrane capacitance, C_{spec}, can be measured exactly, because the diameter of the membrane on the pipette tip can be precisely measured by microscope. Thus, a criterion for the verification of bilayer formation is fulfilled. An advantage of the proposed apparatus is worth mentioning. Conventional plastic pipettes and container are cheap one-way objects and can be easily replaced. No additional expensive devices are necessary for pipette pulling.
(ii) A critical moment in the membrane formation is the relative position of the pipette tip to the water surface. For successful membrane formation the angle between the bath and pipette surfaces was hold at an angle of >90° (see Fig. 2).
(iii) An advantage is the automation of the membrane formation process. As a consequence, the method is especially useful for an user having little experience with the model membrane techniques. The automatic approach allows future extensions to simultaneous formation of several independent membranes, for example, in well plates (see Fig. 5). Conceivably, it enables high throughput screening of ion channels and carriers. Fig. 5 shows a multi-channel set-up 60 comprising a set of devices which are similar to the one depicted in Fig. 1. A set of pipettes tips 62 with electrodes is provided each connected to an amplifier 64. The amplifiers 64 are connected to a respective AD converter 65. A pipette tip elevator 67 is used for raising and lowering each of the pipette tips 62 in a container 61. A Personal Computer 66 is used for operation control and / or data acquisition. There is also provided a multi-channel pipetor 63. Operation of the multi-channel set-up 60 in Fig. 5 is similar to operation of the apparatus according to Fig. 1 which is described in detail above.

Using the advantages of the disclosed teachings, it was established that an increase in the conductivity of UCP2-containing membranes occurs exclusively in the presence of FA such as eicosatrienoic acids (Fig. 3), therefore it can be concluded that FAs are essential for UCP2 function. These acids have previously been shown to activate UCP1 in bilayer membranes (see Urbankova, E. et al.: J. Biol. Chem. 278, 32497-32500 (2003)) and in proteoliposomes (see Zackova, M. et al.: J. Biol. Chem. (2003)).

The reports, concerning the activation of UCP2 by these FAs are contradictory. By ATP addition to the buffer solution on both sides of the membrane the UCP2-mediated conductivity was nearly completely inhibited in the range of studied voltages -50 to +50 mV. The similarity of activation and inhibition patterns of UCP2 to previously described results with UCP1 (see Urbankova, E. et al.: J. Biol. Chem. 278, 32497-32500 (2003)) demonstrates significantly that UCP2 as well might act as an uncoupler. However, to achieve a comparable increase in conductivity a 20-fold higher UCP2 concentration was required. This can be partly explained by not complete refolding of UCP2 from inclusion bodies. The lower uncoupling efficiency of UCP2 raises the question whether UCP2 can generate enough heat for thermogenesis (see Jezek, P. et al.: Physiol Res. 53 Suppl 1, S199-S211 (2004)). Conceivably, the amount of protons transported is able just to attenuate the mitochondrial production of reactive oxygen species. This ability makes this protein an important player in physiology and oxidative stress-related pathologies including atherosclerosis, ischemia-reperfusion damage, inflammation, type-2 diabetes, Parkinson's disease, Alzheimer's disease, and other neurodegenerative diseases.

## Claims

1. A method of forming a lipid bilayer membrane (21), the method comprising:
a) forming a layer (22) of lipid molecules on a liquid surface at an air-liquid interface between a liquid volume and an air volume;
b) moving an aperture (20) from the liquid volume to the air volume through the layer (22) of lipid molecules to form a lipid monolayer membrane (23) at the aperture (20), the aperture (20) being at an end of a bilayer former (3); and
c) moving the aperture (20) from the air volume to the liquid volume through the layer (22) of lipid molecules to form the lipid bilayer membrane (23);
**characterized by** that an angle of >90° is maintained between the liquid surface and a surface of the aperture (20) during the formation of the lipid monolayer membrane (23) and during the formation of the lipid bilayer membrane (21).

2. Method according to claim 1, wherein the aperture (20) is moved from the liquid volume into the air volume by elevating the aperture (20) from the liquid volume into the air volume.

3. Method according to claim 1, wherein the aperture (20) is moved from the air volume into the liquid volume by lowering the aperture (20) from the air volume into the liquid volume.

4. Method according to one of the preceding claims, wherein a space provided inside the former (3) in fluid connection with said aperture (20) is filled with a solution prior to formation of said lipid monolayer membrane (23).

5. Method according to one of the preceding claims, wherein the formation of said lipid bilayer membrane (21) is started when a lower end of the aperture (20) reaches said air-liquid interface.

6. Method according to one of the preceding claims, wherein said formation of said lipid monolayer membrane (23) and said formation of said lipid bilayer membrane (21) are automatically controlled by a control unit (10).

7. Method according to one of the preceding claims, wherein said former (3) is a pipette tip.

8. Method according to one of the preceding claims, wherein said former (3) is made of a plastic material or glass.

9. Method according to one of the preceding claims, wherein said former (3) is a one-way article.

10. Method according to one of the preceding claims, wherein a diameter of a cross section of the aperture (20) is a range from 50 µm to 300 µm.

11. Method according to one of the preceding claims, wherein said formation of said lipid monolayer membrane (23) and said formation of said lipid bilayer membrane are (21) automatically repeated with a repetition rate of about 0.5 Hz to about 2 Hz.

12. An apparatus for forming a lipid bilayer membrane (21), the apparatus comprising:
- a liquid reservoir (2) operable to receive a liquid volume having an air-liquid interface between the liquid volume and an air volume, a liquid surface of the liquid volume having a layer (22) of lipid molecules on the liquid surface;
- a bilayer former (3) with an aperture (20) at one end;
- a mover (4) operable to move the bilayer former (3); and
- a controller (10) operable to control the moving of the bilayer former (3);
where the bilayer former (3) and the mover (4) are operable to move said aperture (20) from said liquid volume into said air volume through said layer (22) of lipid molecules on said liquid surface, thereby forming a lipid monolayer membrane (23) in said aperture (20) and are further operable to move the aperture (20) comprising said lipid monolayer membrane (23) from said air volume into said liquid volume through said layer (22) of lipid molecules on said liquid surface, thereby forming a lipid bilayer membrane (21) in said aperture (20),
**characterized by** that said bilayer former (3) is operable to maintain an angle of >90° between the liquid surface and a surface of said aperture (20) during said formation of said lipid monolayer membrane (23) and during said formation of said lipid bilayer membrane (21).

13. Apparatus according to claim 12, wherein said mover (4) is an elevator.

14. Apparatus according to claim 12 or 13, wherein the bilayer former (3) is a pipette tip.

15. Apparatus according to claim 14, wherein said bilayer former (3) is made of a plastic material or glass.

16. Apparatus according to one of the claims 12 to 15, wherein said bilayer former (3) is a one-way article.

17. Apparatus according to one of the claims 12 to 16, wherein a diameter of a cross section of the aperture (20) is in a range from 50 µm to about 300 µm.

18. Apparatus according to one of the claims 12 to 17, wherein said controller (10) is operable to automatically repeat said formation of said lipid monolayer membrane (23) and said formation of said lipid bilayer membrane (21) with a repetition rate of about 0.5 Hz to about 2 Hz

19. Apparatus according to one of the claims 12 to 18, wherein a second bilayer former (62) is provided, the second bilayer former (62) being connected to a second mover (67) is provided.

## Patentansprüche

1. Verfahren zum Bilden einer Lipiddoppelschicht-Membran(21), wobei das Verfahren die folgenden Schritte umfasst:
a) Bilden einer Schicht (22) von Lipid-Molekülen an einer Flüssigkeitsoberfläche an einer Luft-Flüssigkeitsgrenzschicht zwischen einem flüssigen Volumen und einem Luftvolumen,
b) Bewegen einer Apertur (20) von dem flüssigen Volumen zu dem Luftvolumen durch die Schicht (22) von Lipid-Molekülen, um eine Lipidmonolagen-Membran (23) an der Apertur (20) zu bilden, wobei die Apertur (20) an einem Ende einer Doppelschichtherstellungsvorrichtung (3) angeordnet ist, und
c) Bewegen der Apertur (20) von dem Luftvolumen zu dem flüssigen Volumen durch die Schicht (22) von Lipid-Moleküle, um die Lipiddoppelschicht-Membran (21) zu bilden,
**dadurch gekennzeichnet, dass** ein Winkel von > 90° zwischen der Flüssigkeitsoberfläche und einer Oberfläche der Apertur (20) während des Bildens der Lipidmonolagen-Membran (23) und während des Bildens der Lipiddoppelschicht-Membran (21) eingehalten wird.

2. Verfahren nach Anspruch 1, wobei die Apertur (20) von dem flüssigen Volumen in das Luftvolumen bewegt wird, indem die Apertur (20) von dem flüssigen Volumen in das Luftvolumen gehoben wird.

3. Verfahren nach Anspruch 1, wobei die Apertur (20) von dem Luftvolumen in das flüssige Volumen bewegt wird, indem die Apertur (20) von dem Luftvolumen in das flüssige Volumen abgesenkt wird.

4. Verfahren nach einem der vorangegangenen Ansprüche, wobei eine innerhalb der Doppelschichtherstellungsvorrichtung (3) in Fluidverbindung mit der Apertur (20) angeordnete Aussparung vor dem Bilden der Lipidmonolagen-Membran (23) mit einer Lösung gefüllt wird.

5. Verfahren nach einem der vorangegangenen Ansprüche, wobei das Bilden der Lipiddoppelschicht-Membran (21) gestartet wird, wenn ein unteres Ende der Apertur (20) die Luft-Flüssigkeitsgrenzschicht erreicht.

6. Verfahren nach einem der vorangegangenen Ansprüche, wobei das Bilden der Lipidmonolagen-Membran (23) und das Bilden der Lipiddoppelschicht-Membran (21) mittels einer Steuervorrichtung (10) automatisch gesteuert wird.

7. Verfahren nach einem der vorangegangenen Ansprüche, wobei die Doppelschichtherstellungsvorrichtung (3) eine Pipettenspitze ist.

8. Verfahren nach einem der vorangegangenen Ansprüche, wobei die Doppelschichtherstellungsvorrichtung (3) aus einem Plastikmaterial oder aus Glas gebildet ist.

9. Verfahren nach einem der vorangegangenen Ansprüche, wobei die Doppelschichtherstellungsvorrichtung (3) ein Einwegartikel ist.

10. Verfahren nach einem der vorangegangenen Ansprüche, wobei ein Querschnitt der Apertur (20) einen Durchmesser von 50 µm bis 300 µm aufweist.

11. Verfahren nach einem der vorangegangenen Ansprüche, wobei das Bilden der Lipidmonolagen-Membran (23) und das Bilden der Lipiddoppelschicht-Membran (21) mit einer Wiederholrate von etwa 0,5 Hz bis etwa 2 Hz automatisch wiederholt werden.

12. Vorrichtung zum Bilden einer Lipiddoppelschicht-Membran (21) mit:
- einem Flüssigkeitsreservoir (2), welches konfiguriert ist, ein flüssiges Volumen mit einer Luft-Flüssigkeitsgrenzschicht zwischen dem flüssigen Volumen und einem Luftvolumen aufzunehmen, wobei eine Flüssigkeitsoberfläche des flüssigen Volumen eine Schicht (22) von Lipid-Molekülen an der Flüssigkeitsoberfläche aufweist,
- einer Doppelschichtherstellungsvorrichtung (3) mit einer Apertur (20) an einem Ende,
- einer Bewegungsvorrichtung (4), welche konfiguriert ist, die Doppelschichtherstellungsvorrichtung (3) zu bewegen, und
- einer Steuervorrichtung (10), welche konfiguriert ist, die Bewegung der Doppelschichtherstellungsvorrichtung (3) zu steuern,
wobei die Doppelschichtherstellungsvorrichtung (3) und die Bewegungsvorrichtung (4) konfiguriert sind, die Apertur (20) von dem flüssigen Volumen in das Luftvolumen durch die Schicht (22) von Lipid-Molekülen an der Flüssigkeitsoberfläche zu bewegen, wodurch eine Lipidmonolagen-Membran (23) in der Apertur (20) gebildet ist, und
wobei die Doppelschichtherstellungsvorrichtung (3) und die Bewegungsvorrichtung (4) weiterhin konfiguriert sind, die die Lipidmonolagen-Membran (23) aufweisende Apertur (20) von dem Luftvolumen in das flüssige Volumen durch die Schicht (22) von Lipid-Molekülen an der Flüssigkeitsoberfläche zu bewegen, wodurch eine Lipiddoppelschicht-Membran (21) in der Apertur (20) gebildet ist,
**dadurch gekennzeichnet, dass** die Doppelschichtherstellungsvorrichtung (3) konfiguriert ist, einen Winkel von > 90° zwischen der Flüssigkeitsoberfläche und einer Oberfläche der Apertur (20) während des Bildens der Lipidmonolagen-Membran (23) und während des Bildens der Lipiddoppelschicht-Membran (21) einzuhalten.

13. Vorrichtung nach Anspruch 12, wobei die Bewegungsvorrichtung (4) ein Elevator ist.

14. Vorrichtung nach Anspruch 12 oder 13, wobei die Doppelschichtherstellungsvorrichtung (3) eine Pipettenspitze ist.

15. Vorrichtung nach Anspruch 14, wobei die Doppelschichtherstellungsvorrichtung (3) aus einem Plastikmaterial oder aus Glas gebildet ist.

16. Vorrichtung nach einem der Ansprüche 12 bis 15, wobei die Doppelschichtherstellungsvorrichtung (3) ein Einwegartikel ist.

17. Vorrichtung nach einem der Ansprüche 12 bis 16, wobei ein Querschnitt der Apertur (20) einen Durchmesser von 50 µm bis etwa 300 µm aufweist.

18. Vorrichtung nach einem der Ansprüche 12 bis 17, wobei die Steuervorrichtung (10) konfiguriert ist, das Bilden der Lipidmonolagen-Membran und das Bilden der Lipiddoppelschicht-Membran (21) mit einer Wiederholrate von etwa 0,5 Hz bis etwa 2 Hz automatisch zu wiederholen.

19. Vorrichtung nach einem der Ansprüche 12 bis 18, wobei eine zweite Doppelschichtherstellungsvorrichtung (62) bereitgestellt ist, wobei die zweite Doppelschichtherstellungsvorrichtung (62) mit einer bereitgestellten zweiten Bewegungsvorrichtung (67) verbunden ist.

## Revendications

1. Procédé pour former une membrane d'une double couche de lipides (21), le procédé comprenant :
a) Former une couche (22) de molécules de lipides à une surface d'un liquide sur une couche limite liquide-aérienne entre un volume liquide et un volume aérien,
b) Mouvoir une aperture (20) du volume liquide au volume aérien par la couche (22) de molécule de lipides pour former une membrane d'une seule couche de lipides (23) à l'aperture, l'aperture (20) étant disposée dans une extrémité d'un dispositif de formation (3) d'une double couche, et
c) Mouvoir l'aperture (20) du volume aérien dans le volume liquide par la couche (22) de molécule de lipides pour former la membrane de la double couche de lipides (21),
**caractérisé en ce qu'**un angle de > 90° entre la surface du liquide et une surface de l'aperture (20) est maintenu pendant la formation de la membrane de la seule couche de lipides (23) et pendant la formation de la membrane de la double couche de lipides (21).

2. Procédé selon la revendication 1, **caractérisé en ce que** l'aperture (20) est mû du volume liquide dans le volume aérien en levant l'aperture (20) du volume liquide dans le volume aérien.

3. Procédé selon la revendication 1, **caractérisé en ce que** l'aperture (20) est mû du volume aérien dans le volume liquide en descendant l'aperture (20) du volume aérien dans le volume liquide.

4. Procédé selon l'une des revendications précédentes, **caractérisé en ce qu'**une niche disposée dans le dispositif de formation (3) d'une double couche et étant en connexion fluide avec l'aperture (20) est remplie avec une solution avant la formation de la membrane de la seule couche de lipides (23).

5. Procédé selon l'une des revendications précédentes, **caractérisé en ce que** la formation de la membrane de la double couche de lipides (21) est démarrée, lorsqu'une extrémité inférieure de l'aperture (20) atteint la couche limite liquide-aérienne.

6. Procédé selon l'une des revendications précédentes, **caractérisé en ce que** la formation de la membrane de la seule couche de lipides (23) et la formation de la membrane de la double couche de lipides (21) sont contrôlées automatiquement.

7. Procédé selon l'une des revendications précédentes, **caractérisé en ce que** le dispositif de formation (3) d'une double couche est une pointe d'une pipette.

8. Procédé selon l'une des revendications précédentes, **caractérisé en ce que** le dispositif de formation (3) d'une double couche est composé d'un matériau plastique ou du verre.

9. Procédé selon l'une des revendications précédentes, **caractérisé en ce que** le dispositif de formation (3) d'une double couche est un article jetable.

10. Procédé selon l'une des revendications précédentes, **caractérisé en ce qu'**une coupe transversale comporte une diamètre de 50 µm jusqu'à 300 µm.

11. Procédé selon l'une des revendications précédentes, **caractérisé en ce que** la formation de la membrane de la seule couche de lipides (23) et la formation de la membrane de la double couche de lipides (21) sont répétées automatiquement avec une fréquence d'environ 0,5 Hz jusqu'à environ 2 Hz.

12. Dispositif de formation d'une membrane d'une double couche de lipides (21), comprenant :
- un réservoir (2) pour un liquide, configuré de façon à accueillir un volume liquide avec une couche limite liquide-aérienne entre le volume liquide et un volume aérien, une surface du liquide du volume liquide comportant une couche (22) de molécules de lipides à la surface du liquide,
- un dispositif de formation (3) d'une double couche avec une aperture (20) disposée à une extrémité,
- un dispositif de mouvement (4), configuré de façon à mouvoir le dispositif de formation (3) d'une double couche, et
- un dispositif de contrôle (10), configuré de façon à contrôler le mouvement du dispositif de formation (3) d'une double couche,
le dispositif de formation (3) d'une double couche et le dispositif de mouvement (4) étant configurés de façon à mouvoir l'aperture (20) du volume liquide dans le volume aérien par la couche (22) de molécules de lipides à la surface du liquide, ce qui fait qu'une membrane d'une seule couche de lipides (23) est formée dans l'aperture (20), et le dispositif de formation (3) d'une double couche et le dispositif de mouvement (4) étant en outre configurés de façon à mouvoir l'aperture (20) comprenant la membrane d'une seule couche de lipides (23) du volume aérien dans le volume liquide par la couche (22 ) de molécules de lipides à la surface du liquide, ce qui fait qu'une membrane d'une double couche de lipides (21) est formée dans l'aperture (20),
**caractérisé en ce que** le dispositif de formation (3) d'une double couche est configuré de façon à maintenir un angle de > 90° entre la surface du liquide et une surface de l'aperture (20) pendant la formation de la membrane de la seule couche de lipides (23) et pendant la formation de la membrane de la double couche de lipides (21).

13. Dispositif selon la revendication 12, **caractérisé en ce que** le dispositif de mouvement (4) est un élévateur.

14. Dispositif selon la revendication 12 ou 13, **caractérisé en ce que** le dispositif de formation (3) d'une double couche est une pointe d'une pipette.

15. Dispositif selon la revendication 14, **caractérisé en ce que** le dispositif de formation (3) d'une double couche est composé d'un matériau plastique ou du verre.

16. Dispositif selon l'une des revendications 12 à 15, **caractérisé en ce que** le dispositif de formation (3) d'une double couche est un article jetable.

17. Dispositif selon l'une des revendications 12 à 16, **caractérisé en ce qu'**une coupe transversale comporte un diamètre de 50 µm jusqu'à environ 300 µm.

18. Dispositif selon l'une des revendications 12 à 17, **caractérisé en ce que** le dispositif de contrôle (10) est configuré de façon à répéter automatiquement la formation de la membrane de la seule couche de lipides (23) et la formation de la membrane de la double couche de lipides (21) avec une fréquence d'environ 0,5 Hz jusqu'à environ 2 Hz.

19. Dispositif selon l'une des revendications 12 à 18, **caractérisé en ce qu'**un deuxième dispositif de formation (62) d'une double couche est mis à disposition, le deuxième dispositif de formation (62) d'une double couche étant lié avec un deuxième dispositif de mouvement (67) mis à disposition.
